# EUROPEAN PATENT APPLICATION

(11) **EP 2 676 628 A1**
(43) Date of publication of application: **25.12.2013**
(21) Application number: 13172563.2
(22) Date of filing: 18.06.2013
(51) Int. Cl.: A61B 19/00

(54) **Surgical devices and systems or highlighting and measuring regions of interest**

(30) Priority: 19.06.2012 US 201261661447 P; 29.05.2013 US 201313904126
(71) Applicant: Covidien LP, Mansfield, MA 02048 (US)
(72) Inventor: Durvasula, Ravi, Cheshire, Connecticut CT 06410 (US); Sharonov, Alexey, Bethany, Connecticut 06524 (US); Pinto, Candido Dionisio, Pacifica, California 94044 (US); Liu, Haiying, Winchester, Massachusetts 01890 (US)
(74) Representative: Maschio, Antonio

(57) **Abstract**

The present disclosure relates to systems, devices and methods of highlighting points or regions of interest on a body structure. These systems, devices and methods allow for real-time highlighting of those points or regions that account for repositioning of the viewing instrument (50) that is transmitting the images.

## Description

### CROSS REFERENCE TO RELATED APPLICATION

The present application claims the benefit of and priority to U.S. Provisional Application Serial No. 61/661,447, filed on June 19, 2012, the entire contents of which are incorporated herein by reference.

### BACKGROUND

### Technical Field

The present disclosure relates generally to devices, systems, and methods for locating and measuring characteristics of areas of interest during a surgical procedure, and in particular, devices, systems and methods for use during a minimally invasive surgical procedure for highlighting regions of interest and measuring characteristics of those regions of target surgical sites.

### Background of Related Art

A minimally invasive surgical procedure is one in which a surgeon enters a patient's body through one or more small opening in the patient's skin or a naturally occurring opening (e.g., mouth, anus, or vagina). As compared with traditional open surgeries, minimally invasive surgical procedures have several advantages and disadvantages. Minimally invasive surgeries include arthroscopic, endoscopic, laparoscopic, and thoracic surgeries. Advantages of minimally invasive surgical procedures over traditional open surgeries include reduced trauma and recovery time for patients.

However, some disadvantages of minimally invasive surgery include a lack of direct visualization of the surgical site and reduced dexterity of instruments, as compared to traditional open surgeries. Laparoscopes and other camera-based instruments are often used during a minimally invasive surgery to facilitate visualization of the surgical site. The surgeon must accurately identify and analyze regions of interest within the surgical site that are to be operated upon. To this end, measurements of the regions of interest may be desirable.

Due to accuracy considerations, the complex morphology of the surgical site, and the desirability of keeping the surgical site as sterile as possible, a continuing need exists for non-contact metrology tools.

### SUMMARY

Disclosed herein are devices, systems and methods for highlighting and tracking of points or regions of interest that observed by a viewing instrument during a surgical procedure.

An imaging system for highlighting points and regions of interest of a surgical site is disclosed. The imaging system includes a viewing instrument including a viewing portion, a display to stream images from the viewing instrument in real-time, and an input device to receive a user's input to highlight a point or region of interest on the body structures displayed. The highlighted points or regions of interest of the body structures are configured and adapted to remain highlighted even as the image is transformed in some way either on the display or because of movement of the viewing instrument.

Maintaining the same point or region of interest in a highlighted condition, e.g., an image is superimposed over the point or region of interest, is facilitated by using a tracking system to determine the change in position of the viewing instrument and to correspondingly transform the highlighting or superimposed image to account for the change in angle or position of observation of that point or region of interest. These and other aspects of the disclosure will be described in greater detail in the following detailed description when read in conjunction with the appended figures.

### BRIEF DESCRIPTION OF THE DRAWINGS

Embodiments of the disclosure will be described with reference to the accompanying drawings in which:

Fig. 1A is a front view of the viewing system in accordance with the present disclosure and shown in a body cavity;

Fig. 1B is a perspective view of the viewing system of Fig. 1A shown placed within a seal anchor member that is placed within tissue;

Fig. 2 is a block diagram of an imaging system in accordance with the present disclosure;

Fig. 3A is a screenshot of a surgical site;

Fig. 3B is a transformed image of the screenshot of the surgical site of Fig. 3A;

Fig. 4A is an image shown in a first coordinate system;

Fig. 4B is the image of Fig. 4A and a transformation of that image shown translated;

Fig. 4C is the image of Fig. 4A and a transformation of that image shown rotated; and

Fig. 4D is the image of Fig. 4A and a transformation of that image shown isotropically scaled.

### DETAILED DESCRIPTION

Particular embodiments of the present disclosure will be described herein with reference to the accompanying drawings. In the following description, well-known functions or constructions are not described in detail to avoid obscuring the present disclosure in unnecessary detail. As shown in the drawings and as described throughout the following description, and as is traditional when referring to relative positioning on an object, the term proximal refers to the end of the device that is closer to the user and the term distal refers to the end of the apparatus that is farther from the user.

As will be discussed in detail below, devices, systems, and methods for defining, highlighting, and tracking points or regions of interest on a target site during a surgical procedure in real-time to facilitate consistent and accurate placement of surgical instruments on the target site. Although these devices, systems, and methods are adaptable for use in many surgical procedures, use of these devices, systems, and methods will be discussed with reference to a minimally invasive surgical procedure.

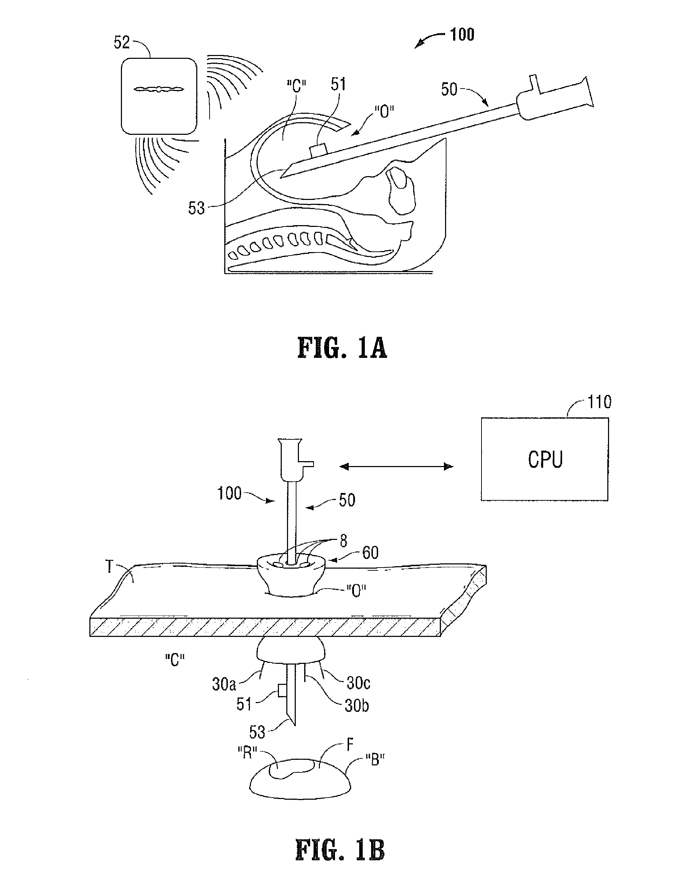

A viewing system 100 will now be described with reference to Figs. 1A-1B. The viewing system 100 includes a viewing instrument 50 that includes a viewing portion 53, e.g., a lens, and a sensor 51 that is configured and adapted to track movement of the viewing instrument 50.

The sensor 51 may be an electromagnetic tracking device that communicates with one or more markers, and may be positioned within or outside of the body cavity "C". For example, the viewing system 100 may be placed within a body cavity "C" to facilitate viewing of underlying body structure "B" and identification of points or regions of interest "R" on the body structure "B". As shown in Fig. 1A, a sensor or marker 52 may be in communication with sensor 51 to facilitate tracking of the viewing instrument 50. One or more markers or sensors may be operatively coupled to the tissue "T" or on another device, e.g., a seal anchor member, that is placed within the body opening "O". For example, as shown in Fig. 1B, markers 30A-30C are operatively coupled to a seal anchor member 60 that is placed within a body opening "O" to access body cavity "C". The seal anchor member 60 includes a plurality of longitudinally extending ports 8 that are adapted for the reception of surgical instruments, e.g., viewing instrument 50, therethrough.

The use of three sensors or markers 30A-30C can facilitate tracking of the sensor 51 through triangulation of the sensor 51 with respect to the three markers 30A-30C. In this way, the position of the sensor 51 and the viewing instrument 50 to which it is coupled may be tracked in real-time. To this end, various tracking devices may be operatively coupled to the viewing instrument 50. Such tracking devices may include electromagnetic, inertial, or optical sensors. A calibration of the surgical viewing instrument 100 can identify an initial position of the viewing instrument 50 relative to the markers 30A-C such that by tracking the change in position, the relative position of the viewing instrument relative to the markers 30A-C is determinable.

In addition, fiducial markers F may be attached to the body structure "B" or elsewhere on the patient's body. The fiducial markers F may be passive (reflecting) or active (emitting). In a passive system, the fiducial markers F may be colored with a color different from the colors of the observed image. As the system 100 identifies the fiducial markers F, the locations of these fiducial markers F can be identified and tracked such that the surgeon is provided with reference points during the surgical procedure, thereby facilitating tracking of nearby structures. Recognition of these fiducial markers F can be accomplished by the surgeon visually or may be automated through the use of a central processing unit (CPU) 110 with which the system 100 is in communication.

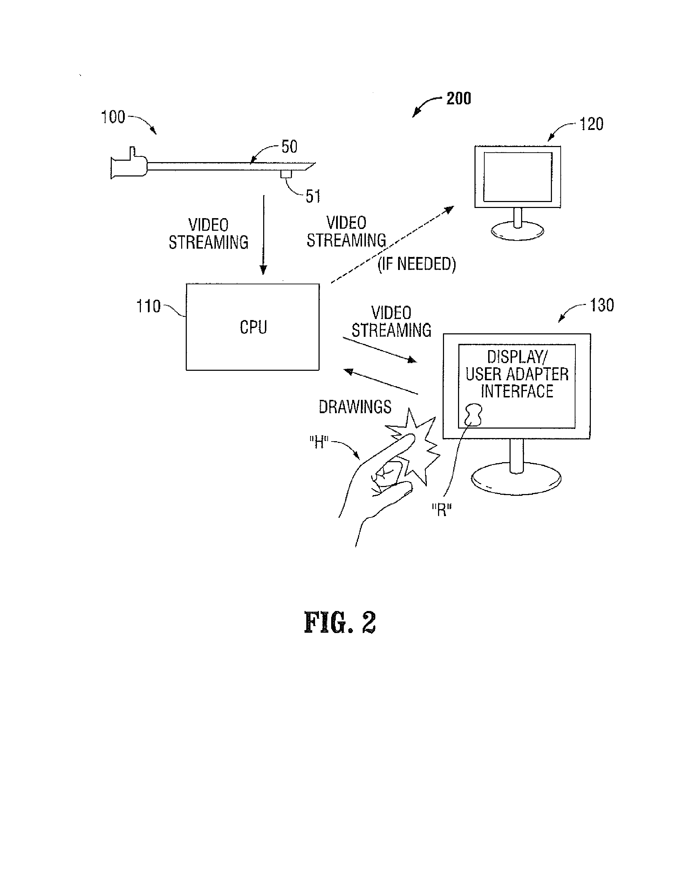

An imaging system 200 will now be described with reference to Fig. 2. The imaging system 200 includes the tracking system 100 to highlight a point or region of interest "R" and to track those highlighted points or areas of interest "R" as the viewing instrument 50 is repositioned. Using the surgical system 200, a surgeon can define, highlight, and track a region of interest "R" during a surgical procedure. To facilitate highlighting, the displayed point or region of interest "R" may be graphically re-colored or encircled or an image may be superimposed upon the point or region of interest "R". Tracking of the viewing instrument 50 facilitates maintaining the highlighting at the same displayed point or region of interest "R" on the body structure "B" even though the viewing instrument 50 is moved and the image appears changed due to the angle or position of observation with respect to the body structure "B".

The imaging system 200 includes the viewing instrument 50, CPU 110, a user interface 130 (which functions as both a display and a user adapter interface), and optionally a remote display 120. The laparoscope 50 is configured and adapted to transmit images or video to the CPU 110. The touch screen display 130 is configured and adapted to both transmit output, e.g., instructions, to the CPU 110, and to receive the images or video that were transmitted to the CPU 110 from the viewing instrument 50. The user interface 130 as shown in Fig. 1 is a touch screen display. However, the user interface 130 may include a separate display and an input device. Input devices may track the inertial motion of the surgeon's hand to encircle the region of interest on the display. Other input devices may use optical, ultrasound, or capacitive means.

As shown in Fig. 2, a region of interest "R" is highlighted on the user interface 130. The highlighting may be a superimposed image or a change in the color of an area of the image. Highlighting of the region of interest "R" can be accomplished by a surgeon using his hand "H" to mark the region of interest "R" on the user interface 130. For example, the user interface 130 may be a touch screen and the surgeon may use his hand "H" to circle the region of interest "R" on the screen with his finger. Alternatively, the region of interest "R" of the displayed image of the surgical site can be encircled using a trackball, mouse, inertial motion device, or another suitable input device.

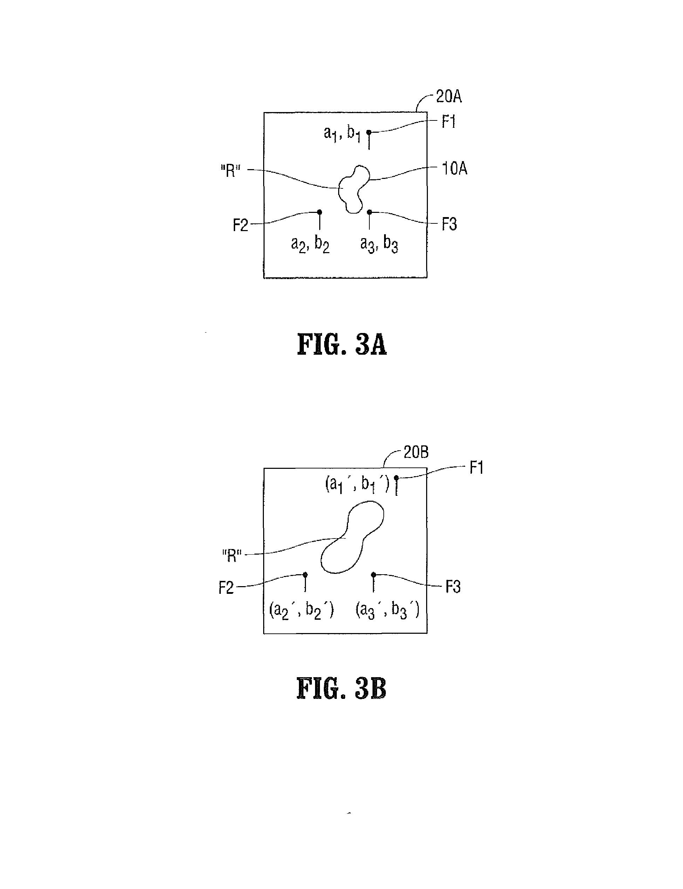

Screenshots displayed on the user interface 130 will now be described with respect to Figs. 3A-B. A first screenshot 20A of the surgical site is shown in Fig. 3A. As shown in Fig. 3A, the region of interest "R" is displayed relative to the fiducial markers F, which are labeled F1-F3, which as discussed above, are configured and adapted to provide relative positioning and tracking information of the viewing instrument 50. The image of the region of interest "R" as shown in the first screenshot 20A defines an identity image 10A that is highlighted. As either the image of the region of interest "R" is reoriented or repositioned, e.g., scaled, translated, or rotated, on a display, due to repositioning the viewing instrument 50, the user interface 130 or the display 120, the perceived dimensional characteristics of the region of interest "R" on the display changes. The tracking of the viewing instrument 50 enables the imaging system 200 to determine the how the image has been transformed such that the relative positioning of the fiducial markers F1-F3 is unchanged and the same region of interest "R" remains highlighted. As shown in Fig. 3A, in a first orientation, the fiducial markers F1-F3 are located on a first coordinate system at points (a1, b1), (a2, b2), and (a3, b3), respectively. In addition to highlighting particular regions, particular points can also be selected and tracked during the course of the procedure. These highlighted points and regions facilitate recognition of those points and regions even when the image appears changed to an observer due to his position or angle relative to those points or areas within the surgical site.

Real-time highlighting of the points and regions of interests "R" on streaming video or images of the surgical site may be accomplished by initially highlighting articular points and regions, and tracking the movements of the image and/or the viewing instrument 50 to transform the highlighted area to account for changes in the position and angle of observation such that the image of the highlighting superimposed on the transmitted image is transformed in the same way as the transmitted image. Therefore, both the highlighting and the transmitted image are transformed, i.e., translated, rotated, or scaled, in the same way such that the position of the point or region of interest "R" remains unchanged relative to the particular body structure "B" that is highlighted. Transformation of the image is determined by tracking the movement of the viewing instrument 50 and altering the highlighted image to account for such movement.

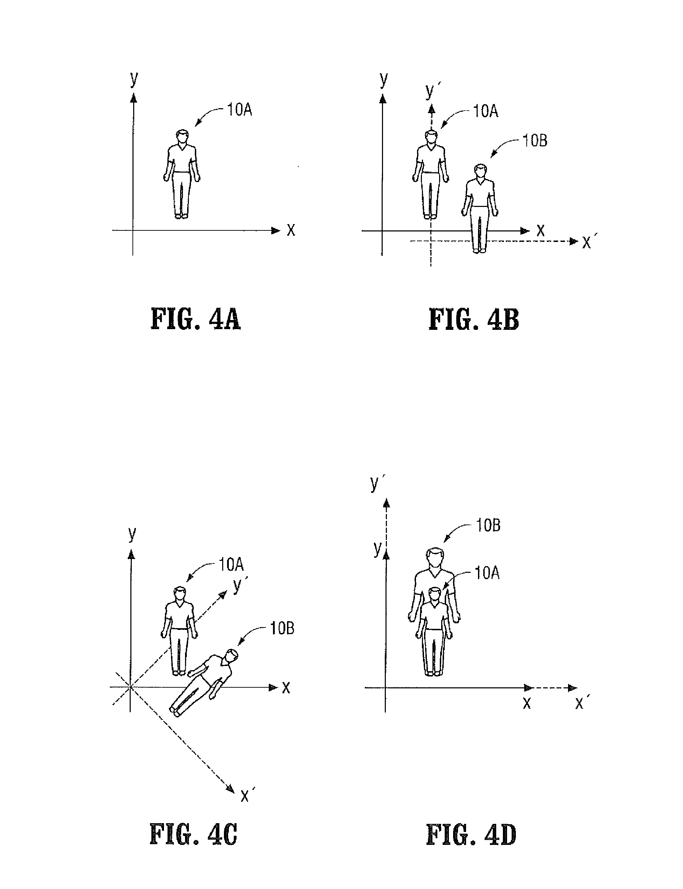

Different transformations of images are shown in Figs. 4A-D. The imaging system 200 first records an identity image 10A (Fig. 4A). Thereafter, the region of interest "R" remains highlighted through means of transformation of the coordinate system such that the highlighted area accurately reflects the contours, angles, and dimensions of the region of interest "R" on the transformed image 10B. Known transformations may change the perspective, rotation, scaling etc. of the image. The image may also be transformed by a light field camera and processor to allow transformations of the image.

A method of using the imaging system 200 to highlight and track a region or point of interest "R" will now be described. The surgeon places the viewing instrument 50 within the body cavity "C" to observe underlying body structures "B" within the body cavity "C" that are displayed on the user interface 130. Once a region of interest has been identified, the clinician or surgeon highlights the area. A suitable input device that is operatively coupled to the system may be used to highlight and delineate the area. For example, as shown in Fig. 2, the user interface is a touchscreen. The surgeon highlights a region of interest "R" on the touchscreen by using his hand "H" to outline the region of interest "R".

The image may be rotated, scaled, or repositioned on a display, and the system 200 will track the regions or points of interest "R" that the surgeon had highlighted such that the same area remain highlighted. In particular, as the viewing instrument 50 is moved, the system 200 will transform the image as necessary such that the same points or regions of interest "R" remain highlighted and can easily be located even after repositioning of the viewing instrument.

It will be understood that various modifications may be made to the embodiments disclosed herein. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. An imaging system comprising:
   a viewing instrument including a viewing portion, the viewing instrument insertable into a body cavity to provide an image of an underlying body structure;
   a display to display images received from the viewing instrument;
   an input device to highlight the images to identify a region of interest on the body structure; and
   a sensor operatively coupled to the viewing instrument to track movement of the viewing instrument, wherein the region of interest remains highlighted as the viewing instrument is repositioned with respect to the body structure.
2. The imaging system of paragraph 1, wherein a second image is superimposed upon the region of interest.
3. The imaging system of paragraph 2, wherein the second image is transformed in real-time to account for movement of the viewing instrument, thereby maintaining highlighting of the region of interest.
4. The imaging system of paragraph 1 further comprising fiducial markers placed on tissue, wherein the fiducial markers are observable by the viewing instrument.
5. The imaging system of paragraph 1 further comprising a seal anchor member, the seal anchor member including markers, the markers in communication with the sensor of the viewing instrument to facilitate tracking of the viewing instrument.
6. A method of highlighting a region of interest within a surgical site comprising:
   providing an imaging system comprising:
      a viewing instrument including a viewing portion;
      a display to display images from the viewing instrument in real-time;
      an input device to superimpose an image on the images transmitted from the viewing instrument to highlight an area of interest; and
      a tracking system including:
         a sensor operatively coupled to the viewing instrument to track movement of the viewing instrument, the image superimposed on the images transmitted from the viewing instrument changing in response to movement of the viewing instrument such that the area of interest remains highlighted;
      placing the viewing instrument within a body cavity;
      observing underlying body structures within the body cavity; and highlighting regions of interest on the display, wherein the regions of interest remain highlighted as the viewing instrument is moved.

## Claims

1. An imaging system comprising:
a viewing instrument including a viewing portion, the viewing instrument insertable into a body cavity to provide an image of an underlying body structure;
a display to display images received from the viewing instrument;
an input device to highlight the images to identify a region of interest on the body structure; and
a sensor operatively coupled to the viewing instrument to track movement of the viewing instrument, wherein the region of interest remains highlighted as the viewing instrument is repositioned with respect to the body structure.

2. The imaging system of claim 1, wherein a second image is superimposed upon the region of interest.

3. The imaging system of claim 2, wherein the second image is transformed in real-time to account for movement of the viewing instrument, thereby maintaining highlighting of the region of interest.

4. The imaging system of any of the preceding claims,further comprising fiducial markers placed on tissue, wherein the fiducial markers are observable by the viewing instrument.

5. The imaging system of any of the preceding claims,further comprising a seal anchor member, the seal anchor member including markers, the markers in communication with the sensor of the viewing instrument to facilitate tracking of the viewing instrument.

6. A method of highlighting a region of interest within a surgical site comprising:
providing an imaging system comprising:
a viewing instrument including a viewing portion;
a display to display images from the viewing instrument in real-time;
an input device to superimpose an image on the images transmitted from the viewing instrument to highlight an area of interest; and
a tracking system including:
a sensor operatively coupled to the viewing instrument to track movement of the viewing instrument, the image superimposed on the images transmitted from the viewing instrument changing in response to movement of the viewing instrument such that the area of interest remains highlighted;
placing the viewing instrument within a body cavity;
observing underlying body structures within the body cavity; and
highlighting regions of interest on the display, wherein the regions of interest remain highlighted as the viewing instrument is moved.
